(19) Europäisches Patentamt
European Patent Office
Office européen des brevets

(11) **EP 3 271 844 B1**

(12) **EUROPEAN PATENT SPECIFICATION**

(45) Date of publication and mention
of the grant of the patent:
**11.10.2023 Bulletin 2023/41**

(21) Application number: **16710819.0**

(22) Date of filing: **09.03.2016**

(51) International Patent Classification (IPC):
***G16H 15/00*** *(2018.01)*

(52) Cooperative Patent Classification (CPC):
**G16H 15/00**

(86) International application number:
**PCT/IB2016/051316**

(87) International publication number:
**WO 2016/147079 (22.09.2016 Gazette 2016/38)**

(54) **GUIDED STRUCTURED REPORTING**

GEFÜHRTE STRUKTURIERTE BERICHTERSTATTUNG

RÉDACTION GUIDÉE DE RAPPORTS STRUCTURÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.03.2015 US 201562135230 P**

(43) Date of publication of application:
**24.01.2018 Bulletin 2018/04**

(73) Proprietor: **Koninklijke Philips N.V.
5656 AG Eindhoven (NL)**

(72) Inventors:
• **SEVENSTER, Merlijn**
**5656 AE Eindhoven (NL)**
• **FORSBERG, Thomas Andre**
**5656 AE Eindhoven (NL)**

(74) Representative: **Philips Intellectual Property &
Standards
High Tech Campus 52
5656 AG Eindhoven (NL)**

(56) References cited:
**US-A1- 2004 024 749      US-A1- 2008 004 505**

**Description**

FIELD

The following relates to patient diagnostic and reporting systems that provide improved structured reports. It finds particular application in conjunction with clinical informatics, especially cardiovascular informatics. However, it is to be appreciated that it will also find application with respect to other usage scenarios, and is not necessarily limited to the aforementioned application.

BACKGROUND

Currently, tools exist, such as the multi-modality tool Philips Xcelera™, which allows clinicians to select report elements and generate a report. A feature of that product is that it allows for reporting in terms of pre-defined finding codes (FCs). Each finding code includes a code component and a textual component. However, this tool requires one-by-one selection of finding codes (FCs), which in turn requires every clinician to navigate multiple menus and submenus prior to obtaining a final report. Generally, once physiological information, including patient imaging data such as echocardiograms, has been generated, clinicians review these images, conduct dictation, and generate a report. However, because the current reporting tools require one-by-one selection of finding codes, this process is time-consuming and may result in reporting errors, such as leaving aspects of a report unaddressed or overlooked. This is because any given structured reporting system might support an average of 1000 different finding codes, while a final report will only include approximately 30 finding codes.

Document US2008004505 further relates to a method and a system for coding medical report documents and for optimizing accurate and compliant coding results.

Thus, a serious problem exists in current technology that results in reporting that is incomplete, tedious, and time-consuming.

SUMMARY

In accordance with aspects of the present invention, a guided structured reporting apparatus and a method for guiding a clinician to address unaddressed aspects of the report are provided as defined in the claims

One advantage of these aspects is that they are compatible with the existing structured reporting systems.

Another advantage resides in improving effectiveness and accuracy of patient reporting.

Another advantage resides in providing a reporting workstation with an improved user interface.

Another advantage resides in providing a reporting workstation facilitating more accurate and rapid reporting.

Another advantage resides in improving the completeness of patient reporting, and ensuring that there are no gaps in the report.

Another advantage resides in improving clinical care outcomes and quality of care.

Another advantage resides in reducing reporting errors and oversights.

Another advantage resides in facilitating the presentation of finding codes and minimizing the need for locating and navigating myriad menus and submenus.

Another advantage resides in providing a guided structured reporting apparatus capable of recommending physiological parameter measurements, such as in order to facilitate patient risk assessment and to ensure unaddressed aspects of a report are addressed.

Still further advantages of the present invention will be appreciated to those of ordinary skill in the art upon reading and understanding the following detailed description. It is to be appreciated that none, one, two, or more of these advantages may be achieved by a particular embodiment.

BRIEF DESCRIPTION OF THE DRAWINGS

The invention may take form in various components and arrangements of components, and in various steps and arrangement of steps. The drawings are only for purposes of illustrating the preferred embodiments and are not to be construed as limiting the invention.

FIGURE 1 diagrammatically illustrates a guided structured reporting apparatus according to one embodiment.
FIGURE 2 illustrates a display of a guided structured reporting apparatus according to one embodiment.
FIGURE 3 diagrammatically illustrates a display of a guided structured reporting apparatus according to another embodiment.
FIGURE 4 diagrammatically illustrates a display on a display device of a guided structure reporting apparatus.

FIGURE 5 illustrates a structured reporting panel according to one embodiment.
FIGURE 6 illustrates a finding code suggestion panel according to one embodiment.
FIGURE 7 illustrates a suggested finding code suggestion panel according to another embodiment.
FIGURE 8 illustrates a narrative report according to one embodiment.
FIGURE 9 illustrates a display of a guided structured reporting apparatus with a narrative report as an element of the display.
FIGURE 10 diagrammatically illustrates a display on a display device of a guided structured reporting apparatus with a narrative report as an element of the display.
FIGURE 11 illustrates a method flow chart or means diagram for the guided structured reporting of physiological information by using a guided structured reporting apparatus.

DETAILED DESCRIPTION

The present disclosure is directed to apparatuses and computer-implemented methods for the guided structured reporting of patient physiological information which in various embodiments provide various advantages such as reducing reporting errors, guiding the clinician to address potentially overlooked aspects of a patient report, and improving efficiency of the reporting workstation user interface.

The present disclosure is further directed to apparatuses and computer-implemented methods generating and suggesting physiological parameters to be considered and adopted into a report.

The present disclosure is further directed to apparatuses and computer-implemented methods incorporating and displaying suggested physiological parameters to be considered and adopted into a report that reduces the burden placed on clinicians and other users of the apparatuses and methods when navigating interface the disclosed apparatuses and methods.

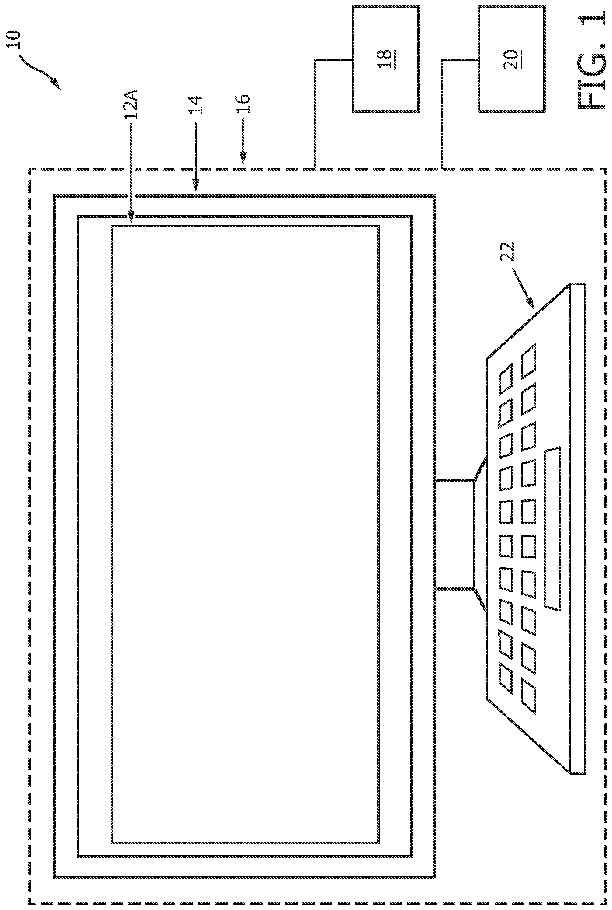

FIGURE 1 diagrammatically illustrates a guided structured reporting apparatus **10** according to one embodiment. According to this embodiment, the apparatus **10** includes a workstation**16** and one or more processors **18** connected to the workstation **16**. The workstation includes a display device **14** configured to display physiological information, and at least one user input device **22** (e.g., the illustrative keyboard, and/or a mouse or other pointing device, and/or a touch soverlay on the display **14**, or so forth) configured to receive one or more inputs. The one or more processors **18** connected to the workstation **16** are configured to at least: receive physiological information; receive one or more inputs from the input **22**; generate a display **12A** presented on the display device **14**; generate one or more suggested finding codes **48**; and update the display **12A** on the display device **14** based on inputs from the input device **22** or in response to receiving additional physiological information. It will be appreciated that the one or more processors **18** may be separate from the user interfacing components **14**, **22** (e.g. a web-based server or cloud computing system) and/or may be integrally built (e.g. a notebook or desktop computer in which a processor is integral to the computer) Additionally, in some embodiments, the workstation **16** of the apparatus **10** can be connected to at least one memory system **20** in which patient information, including physiological information is stored. The memory **20** can also store one or more suggested finding codes and newly created patient reports. In some embodiments, the memory **20** includes a clinical information system.

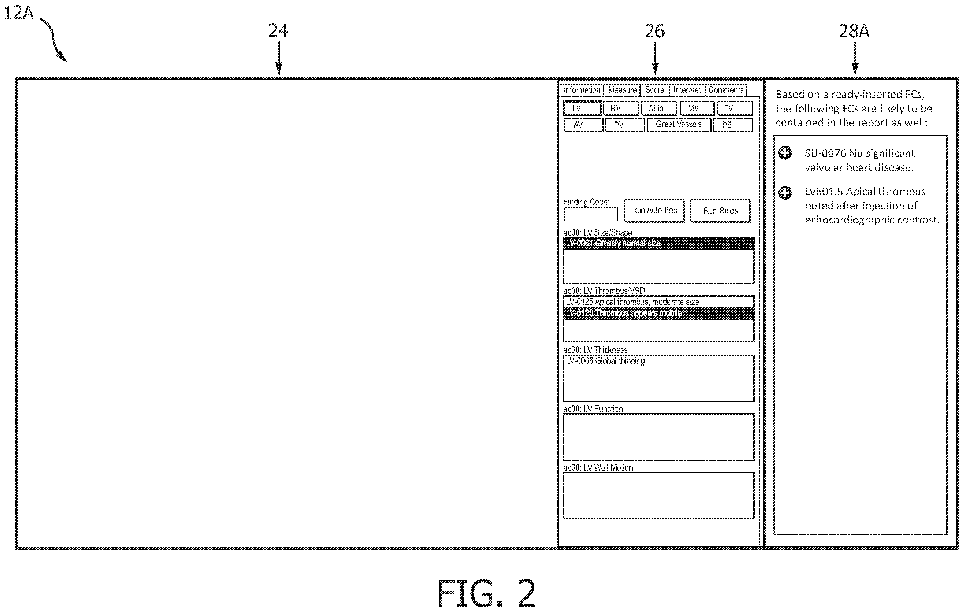

FIGURE 2 illustrates an enlarged view of the display **12A** of FIGURE 1. As seen in FIGURE 2, the one or more processors **18** of the apparatus **16** are configured to generate the display **12A** including at least a structured reporting panel **26** and a finding code suggestion panel **28A**. The display **12A** also includes an imaging panel **24** (or, more generally, a patient data presentation or rendering or summarization pane) in which additional patient physiological information, such as echocardiogram images, can be reviewed. In particular embodiments, the finding code suggestion panel **28A** may be a persistent window. In other words, in particular embodiments, the finding code suggestion panel **28A** may always be an element of the display **12A** in the apparatus **16**.

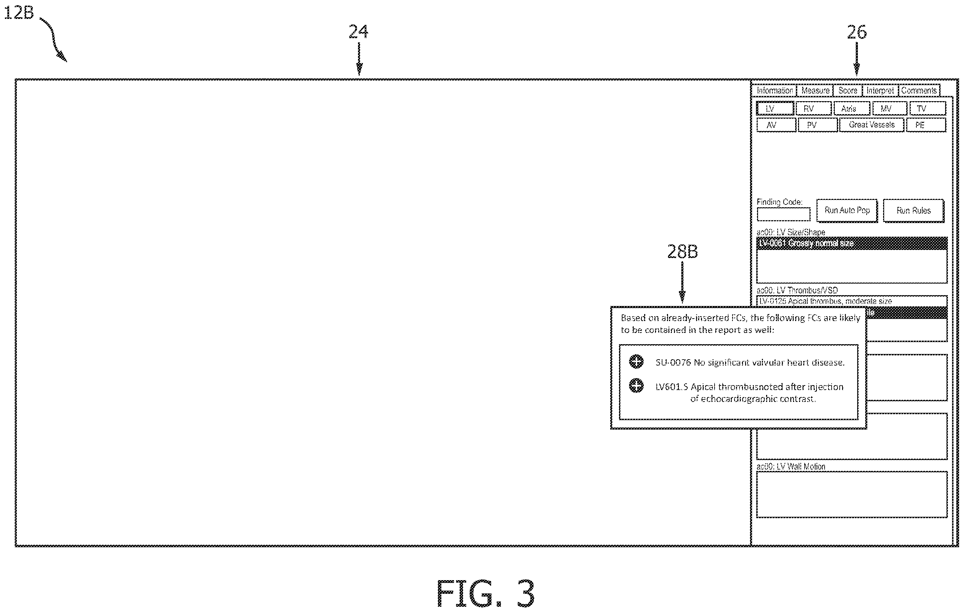

In alternative embodiments, as seen in FIGURE 3, the one or more processors **18** of the apparatus **16** are configured to generate a display **12B** on the display device **14** including at least a structured reporting panel **26** and a finding code suggestion panel **28B**, wherein the finding code suggestion panel **28B** is not a persistent window. In other words, in particular embodiments, the finding code suggestion panel **28B** may be a transient window, and only be generated in the display **12B** after receiving some input from the user via the one or more user input devices **22**.

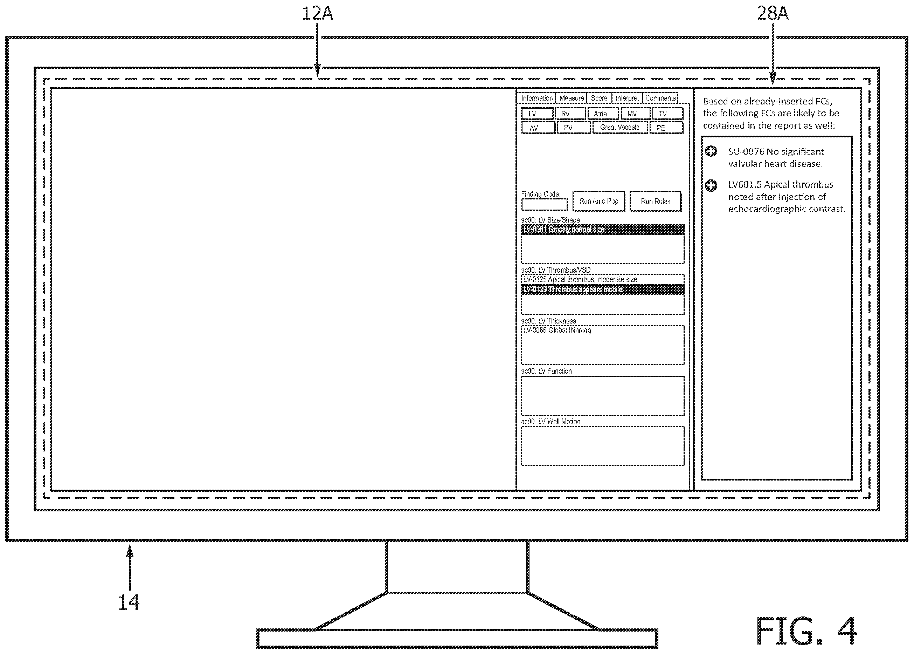

As seen in FIGURE 4, the one or more processors **18** of the apparatus **16** are configured to generate the display **12A** on the display device **14** of the apparatus **10**. In particular embodiments, the display **12A** may be a display **12A** as seen in FIGURES 2 and 3, while in other embodiments, the display **12A** may not include an imaging panel **24**.

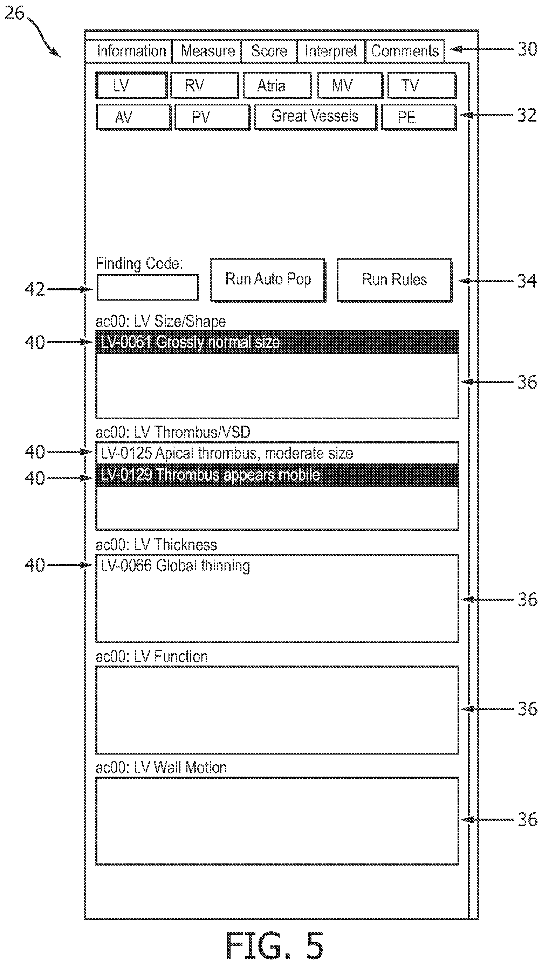

FIGURE 5 illustrates a structured reporting panel **26** according to one embodiment. In particular embodiments, the structured reporting panel **26** may include an anatomical submenu **32** and aspect submenus **36**. An anatomical submenu **32** and an aspect submenu **36** may include a variety of options depending on the particular application of the invention. In particular embodiments, such as cardiovascular applications, an anatomical submenu **32** may include options for "LV" (left ventricle), "RV" (right ventricle), "Atria", "MV", "TV", "AV", "PV", "Great Vessels", and "PE". In particular embodiments, such as cardiovascular applications, an aspect submenu **36** may include aspects such as "Size/Shape", "Thrombus/VSD", "Thickness", "Function", and "Wall Motion". In particular embodiments, the aspect submenu **36** options may vary across different anatomical submenu **32** options, and thus may change depending on which anatomical submenu **32** option is currently displayed.

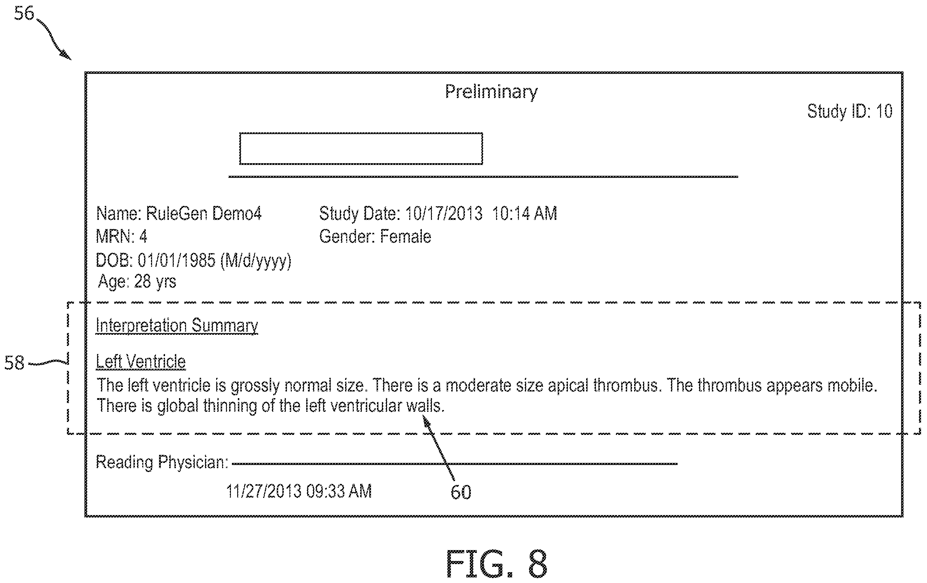

In particular embodiments, as seen in FIGURE 5, the structured reporting panel **26** may also include one or more finding code generation buttons **34**, one or more drop-down menus, and an option to search for finding codes **42**. In particular embodiments, as also seen in FIGURE 5, the structured reporting panel **26** preferably also includes one or more adopted finding codes **40**. Adopted finding codes **40** are used to generate a narrative report **56** (e.g. FIGURE 8).

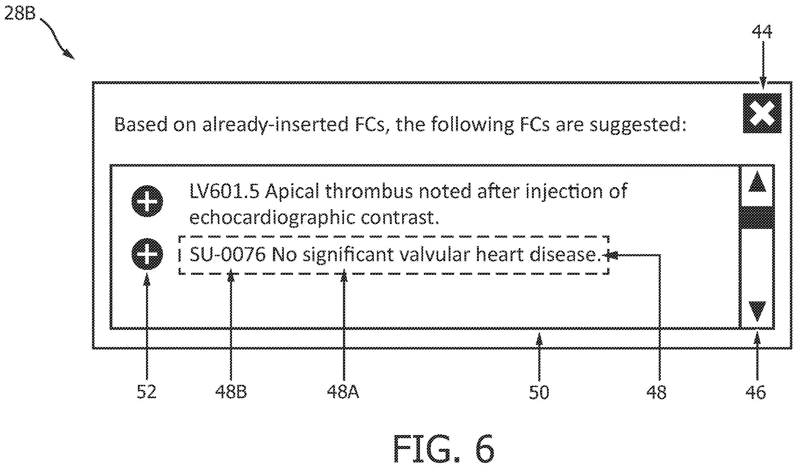

FIGURE 6 illustrates a drop-down menu finding code suggestion panel **28B** according to one embodiment. In particular embodiments, a finding code suggestion panel **28B** may include one or more suggested finding codes **48** and one or more adoption buttons **52** corresponding to the one or more suggested finding codes **48**. In particular embodiments, the finding code suggestion panel **28B** may be a transient suggestion panel, which is only generated by the one or more processors **18** on the display **12B** after receiving an input from an input **22**. In particular embodiments, the finding code suggestion panel **28B** may have an exit or close button **54**, wherein upon receiving an input from the input **22**, the one or more processors **18** removes the suggestion panel **28B** from the display. In other words, in particular embodiments, a display **12B**, contains a finding code suggestion panel **28B** even if the finding code suggestion panel **28B** is not always visible or displayed in the display **12B**.

In particular embodiments, the suggested finding codes **48** and adoption buttons **44** may be contained in a sub-window **50** of the suggestion panel **28B**. Additionally, in particular embodiments, the finding code suggestion panel **28B** may contain a scroll-bar **46**, wherein the one or more processors **18** are configured to scroll through lists of suggested finding codes **48** within the finding code suggestion panel **28B** upon receiving input from the input **22**.

In particular embodiments, each finding code **40** and suggested finding code **48** may include a textual component **48A** and a code component **48B**. Finding codes **40**, **48** may be at least one of several different types of finding codes. In particular embodiments, a finding code **40**, **48** may be at least one of: a binary statement finding code, which are either true or false (e.g., "The LV is severely dilated."); a multiple-choice statement finding code, in which a clinician or other user may select one of several predetermined options (e.g, "Mitral valve area by pressure half-time is..." with options to select for "< 100 msec", "between 100 msec and 180 msec", and "greater than 180 msec"); and a measurement statement finding code, in which a manually created measurement is used to complete a evaluation statement regarding a particular anatomical aspect.

As seen in FIGURE 6, one or more processors **18** of the apparatus **10** may be configured to generate and display one or more suggested finding codes **48** for a variety of anatomies and aspects of anatomies. In other words, in some embodiments, the finding code suggestion panel **28B** may include suggested finding codes **48** that are related to and not related to the currently displayed anatomical submenu.

In the following, two illustrative embodiments are described that are suited for suggesting finding codes **48**. The first is most suitable in clinical settings where large amounts of physiological information, such as from a database of structured reports for different patients, are not available. The second is most suitable in clinical settings where large amounts of physiological information, such as from a databased of structured reports for different patients, is available. However, these embodiments are not limited to such situations and may be performed in many different clinical environments. Moreover, the two illustrative embodiments may occur concurrently.

Furthermore, according to the present disclosure, either or both of the illustrative embodiments are able to (1) provide finding code suggestions **48** that are implied by the finding codes **40** contained in the current report, and/or (2) provide finding code suggestions **48** that are most informative given the finding codes **40** contained within the current report. Thus, either or both of the illustrative embodiments may allow for the adoption of overall impression statements (i.e., suggested finding codes **48**) given concrete finding codes **40** already contained within the current report, and may allow for the detection of anatomies **32** and aspects **36** that require further completion (e.g., if a clinician or other user of the apparatus **10** has fully specified all aspects **36** of the left ventricle anatomy **32**, the illustrative embodiments will suggest finding codes **48** for adoption in anatomies **32** and aspects **36** that have not be addressed).

The foregoing displays are generated by a combination of a current report contents demon in the form of an engine that tracks all finding codes contained in the current study. This demon updates every time the contents of the report change. A finding code engine suggests the one or more finding codes for adoption. The user interface engine presents the one or more suggested finding codes to the user as shown in the displays discussed above. The current report demon can be implemented using standard application programming interface (API) methods. The finding code suggestion engine can be provided in various ways. In one embodiment, the finding code suggestions are selected based on the finding codes contained in the current (preliminary) report that the clinician is currently preparing. In another embodiment, the finding codes that are most important relative to the finding codes contained in the current (preliminary) report are suggested.

The first embodiment allows the insertion of overall impression statements given concrete findings. The second embodiment detects what aspects need further attention. For example, if the user has fully specified all values regarding the organ being analyzed. The system will automatically start suggesting the adoption of finding codes in areas that have not been addressed. For example, if all the values regarding the left ventricle have been fully specified, the system will start suggesting the adoption of finding codes in other areas that have not been addressed, such as the right ventricle.

When used in unison, these two embodiments can drive the entire structured reporting process so as to minimize the need for locating and navigating to sub-windows in which new finding codes need to be entered. The second type of functionality searches for the finding codes that carry the most informational value and suggest such codes in order to assist the clinician in covering all aspects of the exam's interpretation. The first type of functionality searches for finding codes that are implemented by the finding codes that have already been entered. The combination of these two functionalities facilitates avoiding gaps in the report.

According to the invention, the finding codes are suggested by at least one of a rule-driven implementation in which a set of background rules are used for making suggestions, or an implementation, in which the finding code suggestions are data driven. Suggestions are made based on statistics or other types of values derived from a database of prior reports.

The first illustrative embodiment pertains to a rule-driven implementation of suggesting finding codes **48** for display in a guided structured reporting apparatus **10**. Generation of the suggested finding codes **48** can be performed by using a set of background rules used for making suggestions. According to this illustrative embodiment, the set of rules models the correlations between pre-existing finding codes. For example, a set of rules might be summarized according to the following:

*If* {**at least one, all**} *of the finding codes* $[A_1, A_2, \ldots, A_n]$ *are in the current report* **R**, *then suggest* {**at least one, all**} *of the finding codes* $[S_1, S_2, \ldots, S_n]$ *for adoption in the current report* **R**.

According to the first illustrative embodiment, the set of background rules used for generating suggested finding codes **48** as described above contains quantifier values for the finding codes available, including any pairwise combinations of finding codes, and evaluates determines whether the existing finding codes **40** imply other finding codes that should be suggested **48**. These quantifier values may not be known, but may be estimated by retrospectively analyzing past patient data, and may be fine-tuned manually.

The second illustrative embodiment pertains to a data-driven implementation of suggesting finding code **48** for display and for adoption in a guided structured reporting apparatus **10**. Generation of the suggested finding codes **48** can be performed by examining retrospective physiological information to determine the most similar prior sets of finding codes **40** and the likely next entered finding code. This process is expedited by determining the information value of potential finding codes such that high information value finding codes are suggested and used in the report. In some embodiments, one or more processors **18** are configured to generate suggested finding codes **48** by examining contents of the memory system **20** such as a database of retrospective echocardiogram reports (e.g. electrocardiogram reports that have been prepared for past patients), and suggest finding codes **48** that always occur (or frequently occur, e.g. above some specified threshold) given that another set of one or more finding codes **40** occur. In one approach, every finding code is regarded as a random variable that can take values from a discrete and finite domain: binary statement finding codes take values from the domain {true, false}; multiple-choice finding codes take values from their set of options; and measurement finding codes take values from the domain {25th percentile, 50th percentile, 75th percentile}.

Thus, according to the second illustrative embodiment, if the current report contained finding codes $[A_1, A_2, \ldots, A_n]$ **40**, then the one or more processors **18** will generate suggested finding codes [B] **48** for which the conditional probability:

$$P(B|A_1, \ldots, A_n) \geq \theta$$

for a threshold, $\theta$. To ensure the significance of the relation, particular embodiments may impose that the number of reports containing the set of finding codes $[A_1, A_2, \ldots, A_n]$ **40** and [B] exceeds a certain fixed or statistically determined threshold.

In particular embodiments of the second illustrative embodiment, the one or more processors **18** may generate suggested finding codes **48** based on the information value, or conditional entropy, of a finding code [B] given existing finding codes $[A_1, A_2, \ldots, A_n]$ **40**. For example, in some embodiments of the second illustrative embodiment, the one or more processors **18** may generate suggested finding codes **48** by excluding finding codes that have a low information value based on the current finding codes **40** according to the following:

$$H(B|A_1, \dots, A_n) \leq \chi$$

for a threshold $\chi$, where H is the conditional entropy of the finding code B given the set of adopted finding codes [$A_1$, $A_2$,..., $A_n$] **40**. If the information value of B is below $\chi$, then the finding code B carries little information per se and/or given the values of existing finding codes [$A_1$, $A_2$,..., $A_n$] **40**. In other embodiments, the one or more processors **18** may generate suggested finding codes **48** by determining whether a finding code has a high information value given the finding codes **40** currently contained in the report according to the following:

$$H(B|A_1, \dots, A_n) \leq \lambda$$

for a threshold, $\lambda$.

According to either illustrative embodiment, the one or more processors **18** may update the one or more suggested finding codes **48** every time the contents of the report (i.e., the adopted finding codes **40**) are altered. Additionally, the one or more processors **18** may be configured in either illustrative embodiment to sort and display the list of suggested finding codes **48** based upon the suggestion code's probability or conditional entropy. In particular embodiments, the one or more processors **18** may limit the number of suggested finding codes **48** displayed or generated at one time. In particular embodiments, the one or more processors **18** may be configured to display only those suggested finding codes **48** that are anatomically related to the anatomical region currently displayed. For example, in some embodiments, the finding code suggestion panel **28A** may only contain suggested finding codes **48** anatomically related to the left ventricle if "LV" is currently displayed in the structured reporting panel **26**, as seen in FIGURE 5.

The user interface engine preferably presents the suggested finding codes to the user in unambiguous, uncluttered ways. In one embodiment, the user interface engine presents the suggestions in a separate panel as a pop-up or background window **28B** (see FIGURE 3) in which the suggestions are refreshed every time the contents of the report are altered. In the other embodiment, the suggested finding codes are displayed in a native structured reporting window (e.g. window **28A** of FIGURE 2) for selection. These approaches provide the clinician with suggestions, but do not actually modify the report being prepared by the clinician - in other words, the clinician is solely responsible for the content of the report, while the suggestions provided in the unambiguous window **28A** or **28B** are assistive in nature.

When there are multiple finding codes, the interface optionally sorts them based on the probability and/or entropy of the scores found by the binding code suggestion engine, in order to display the "most likely" finding codes at the top of the list or otherwise emphasized. If the number of suggested finding codes is too large to fit into the window **28A** or **28B**, the interface engine can clip off (i.e. not display) the least probable end of the list.

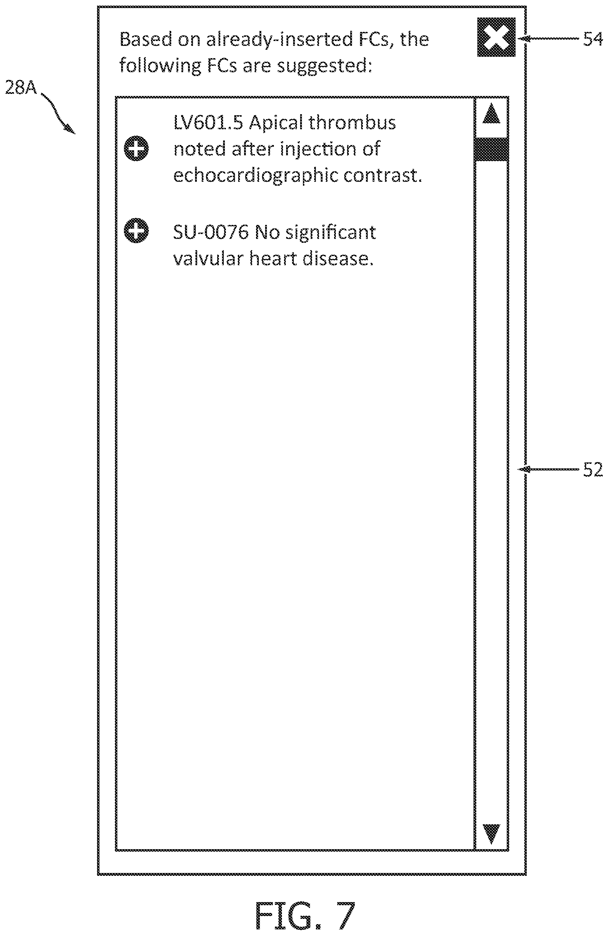

The user interface can also explain the mode of functionality that is supported by the suggestion (that is, the rationale by which the suggestion is being made). In the first functionality category, the interface could communicate this mode with a prefix such as "based on the already inserted finding codes, the following finding codes are likely to be contained in this report as well." In the second functionality category, the interface could communicate with a prefix such as: "please specify the status of finding code A, which is optimally clinically informative according to our analysis." In a more advanced embodiment, this engine can display only the suggested finding codes that are anatomically related, i.e., can be selected in the anatomical window, e.g., left ventricle as illustrated in FIGURE 7.

FIGURE 7 illustrates an enlarged view of the illustrative suggested finding code panel **28A**. In particular embodiments, the finding code suggestion panel **28A** may be a persistent suggestion panel, as seen in FIGURE 2. As seen in FIGURE 7, one or more processors **18** are configured to generate the persistent suggestion panel **28A** that, unlike the transient suggestion panel **28B** in FIGURE 6, may always be displayed in the display **12A**.

FIGURE 8 illustrates a narrative report according to one embodiment. In particular embodiments, the one or more processors **18** operating in conjunction with the display device **14** and the one or more user interface devices **22** are configured to enable the clinician to generate a narrative report **56** based on the finding codes **40** that have been adopted into the current report. In particular embodiments, a narrative report contains an interpretation summary section **58**, in which one or more anatomies **32** may be summarized in sub-sections **60**. In particular embodiments, the one or more processors **18** display only the textual components of the adopted finding codes **48** within the interpretation summary section **58** and the anatomical sub-sections **60**.

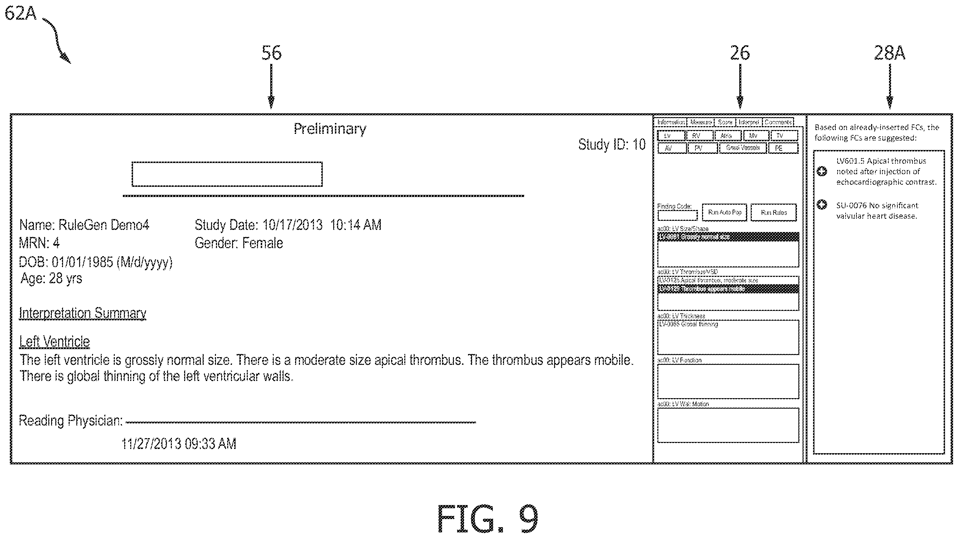

FIGURE 9 illustrates a display of a guided structured reporting apparatus with a narrative report as an element of the display. As seen in FIGURE 9, the one or more processors may be configured to generate a display **62A** in which a narrative report **56** is displayed. In some embodiments, the narrative report **62A** may be displayed rather than displaying an imaging panel **24**. In other words, the narrative report **56** may be displayed even if an imaging panel **24** is not also displayed. However, like in FIGURES 2 and 3, the display **62A** may include a structured reporting panel **26** and a persistent suggestion panel **28A** (as shown in FIGURE 9) or a transient suggestion panel **28B** (e.g. see FIGURE 3).

In another embodiment, instead of a separate window for the suggested field codes (either attached or free floating), the suggested field codes are automatically inserted in the report being prepared, such as by filling in boxes in the report. To denote that the field codes are suggested, they are "greyed out", semitransparent, or otherwise visually denoted as suggested rather than adopted. If the clinician wants to adopt one of the greyed out suggested field codes, double-clicking the suggested field code adds it to the report in the same text format as the other adopted field codes. In one variation on this embodiment, there is an upper limit to the number of greyed out or suggested field codes which are presented to avoid overwhelming the clinician, e.g. 2 field codes per panel. More specifically, the 2 most relevant (by any chosen metric) suggested field codes appear greyed out. The clinician can easily double click on the one(s) to be added to the report. Once one suggested field code is adopted, a new greyed out suggested field code is inserted in the place in the report appropriate to the new field code to replace the adopted field code.

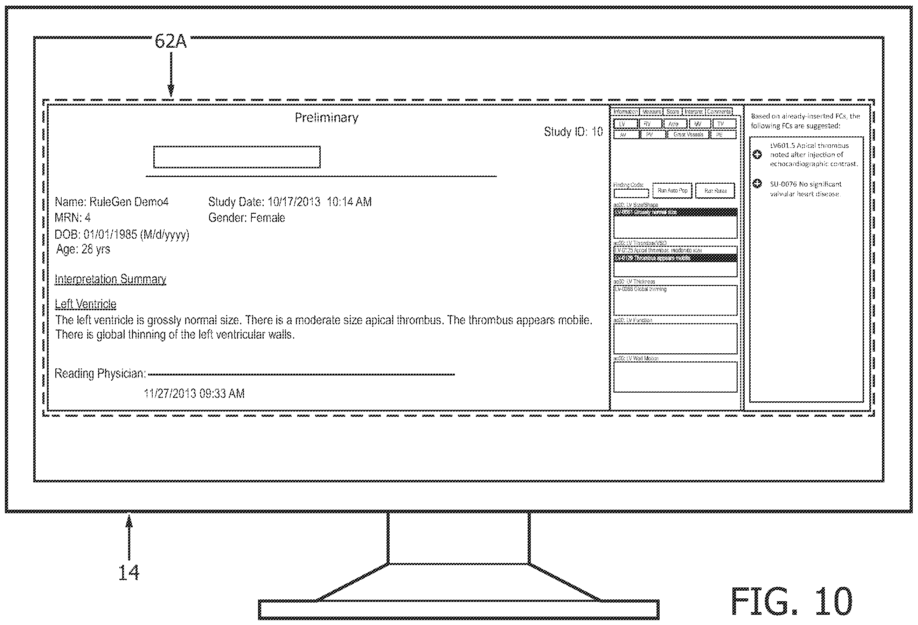

FIGURE 10 diagrammatically illustrates a display on a display device of a guided structured reporting apparatus with a narrative report as an element of the display. In particular embodiments, the one or more processors may generate a display **62A** including a narrative report **56** on a display device **14** of the apparatus **10**.

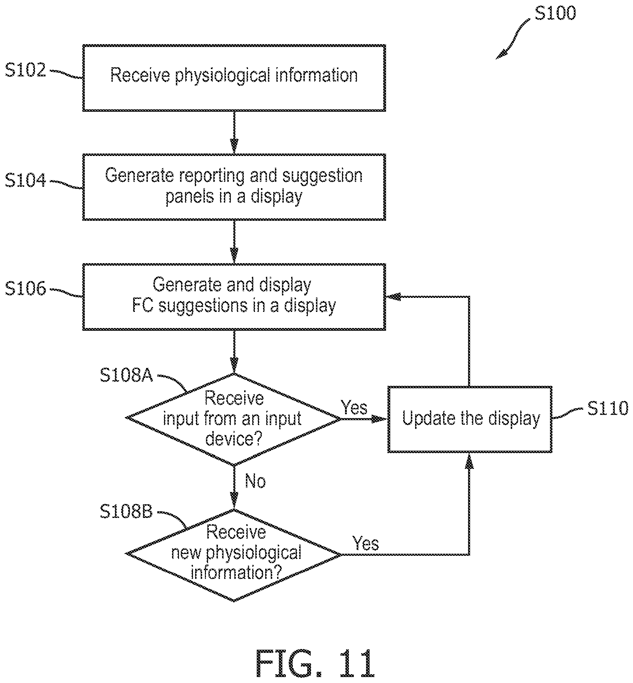

FIGURE 11 illustrates a method **S100** flow chart for the guided structured reporting of physiological information by using a guided structured reporting apparatus. In a first step **S102**, physiological information is received at a workstation **16**. In particular embodiments, the physiological information may include at least one of patient physiological imaging information, patient records, patient finding codes **48**, patient structured reports **58**, and stored rules for suggesting finding codes. Using this physiological information, the one or more processors **18** generate a display **12, 62** on the display device **14** of the workstation **16** in a second step **S104.** In particular embodiments, the display may include at least one of a structured reporting panel **26** and a finding code suggestion panel **28**. In some embodiments, as described above, a transient suggestion panel **28B** may always be visible. In a third step **S106**, finding code (FC) suggestions are generated by the one or more processors as described herein based on the physiological information. As the clinician prepares the report, he or she inputs data in step **S108A**, in which the one or more processors **18** receive user input via the user input device(s) **22**, for example in the form of keyboard entry, selections using a mouse or other pointing device, or so forth. In a forth step **S108B**, the one or more processors **18**, which are configured to receive physiological information, may receive new physiological information. In particular embodiments, new physiological information may include new patient imaging data. In step **S110**, the one or more processors **18** update the display **12/62** based upon the input received in the third step **S108A** or the information received in the forth step **S108B**. According to the invention, updating the display includes generating and displaying a narrative report based upon the finding codes **40** adopted by the clinician in the current report, and may include adopting one or more finding codes **48** by the clinician from the finding code suggestion panel **28** into the structured reporting panel **26**. Optionally, in some embodiments, the one or more processors may generate and display new suggested finding codes **48** after updating the display **12/62** in the fifth step **S110**, and the preceding steps are repeated.

The invention has been described with reference to the preferred embodiments. Obviously, modifications and alterations will occur to others upon reading and understanding the proceeding detailed description. It is intended that the invention be construed as including all such modifications and alterations insofar as they come within the scope of the appended claims thereof.

**Claims**

1. A guided structured reporting apparatus (10) for guiding a clinician to address unaddressed aspects of a report, the apparatus comprising:

   a workstation (16) including a display device (14) configured to display physiological information, and an input (22) configured to receive one or more inputs from a clinician;
   one or more computer processors (18) connected to the workstation (16), the one or more computer processors (18) being configured to:

      receive physiological information;
      generate and display a preliminary report on the display device (14);
      receive and display finding codes (40) from the input (22) selected by the clinician for inclusion in the preliminary report;
      generate one or more suggested finding codes (48) using the received physiological information, by at least one of a rule-driven implementation, in which a set of background rules are used for making suggestions, the set of rules modeling the correlations between pre-existing finding codes (40) and a data-driven implementation, in which statistics or other types of values derived from a database of prior reports are used for making suggestions by examining retrospective physiological information to determine the most similar prior sets of finding codes (40) and the likely next entered finding code;

display the one or more suggested finding codes for the clinician to select among;

receive a clinician's selection of one or more of the displayed finding codes; and

update the displayed finding codes on the display device (14) including generating and displaying a narrative report (56) based on at least one of the clinician selected finding codes (40, 48) and physiological information.

2. The apparatus (10) according to claim 1, further including at least one memory (20) configured to store finding codes (48).

3. The apparatus (10) according to claim 1, wherein the display (12A) includes:

a structured reporting panel (26); and
a finding code suggestion panel (28A/28B).

4. The apparatus (10) according to claim 3, wherein the finding code suggestion panel (28A/28B) includes one of a persistent finding code suggestion panel (28A) and a transient finding code suggestion panel (28B).

5. The apparatus (10) according to claim 3, wherein the finding code suggestion panel (28A/28B) includes:

one or more suggested finding codes (48); and
one or more adoption buttons (52) corresponding to the one or more suggested finding codes (48) by which the clinician selects one or more of the suggested finding codes.

6. The apparatus (10) according to claim 1, wherein the one or more suggested finding codes (48) include a code component (48A) and a textual component (48B).

7. The apparatus (10) according to claim 1, wherein the one or more suggested finding codes (48) include at least on of a binary statement finding code, a multiple-choice statement finding code, and a measurement statement finding code.

8. The apparatus (10) according to claim 3, wherein the structured reporting panel (26) comprises:

one or more anatomical submenus (32);
one or more aspect submenus (36);
one or more drop-down menus;
one or more suggested finding code generation buttons (34); and
one or more adopted finding codes (40).

9. The apparatus (10) according to claim 1, wherein the one or more processors (18) are configured to receive physiological information includes at least one of patient physiological imaging information, patient records, patient finding codes (48), patient structured reports (58), and stored finding code patterns.

10. The apparatus (10) according to claim 3, wherein the display (12A/62A) further comprises an imaging panel (24).

11. The apparatus (10) according to claim 10, wherein the preliminary report is a structured report including one or more anatomical summaries (60);
wherein the anatomical summaries (60) include the textual components (48A) of the selected finding codes (40) from a structured reporting panel (26).

12. A computer-implemented method for guiding a clinician to address unaddressed aspects of a report, the method comprising:

receiving (5102) physiological information at a workstation (10);
generating (S104) and display a preliminary report on a display device (14) using one or more processors (18);
receiving and displaying finding codes (40) from the input (22) selected by the clinician for inclusion in the preliminary report;
generating (S106) one or more suggested finding codes (48) using the received physiological information, by at least one of a rule-driven implementation, in which a set of background rules are used for making suggestions, the set of rules modeling the correlations between pre-existing finding codes (40) and a data-driven implementation, in which statistics or other types of values derived from a database of prior reports are used for making suggestions by examining retrospective physiological information to determine the most similar prior sets of finding codes (40) and the likely next entered finding code;

displaying (S106) the one or more suggested finding codes (48) for the clinician to select among using one or more processors (18);

receiving a clinician's selection of one or more of the displayed finding codes from a user input (22); and

updating the displayed finding codes including generating and displaying a narrative report (56) based on the one or more of the clinician selected finding codes and physiological information.

13. The method (S100) according to claim 12, wherein generating the suggested finding codes (48) includes generating a code component (48B) and a textual component (48A).

14. A non-transitory computer readable medium (20) carrying software for controlling one or more processors (18) to perform the method according to claim 12.

## Patentansprüche

1. Angeleitetes strukturiertes Berichtsgerät (10), um einen Arzt dazu anzuleiten, unbehandelte Aspekte eines Berichts zu behandeln, das Gerät umfassend:

eine Arbeitsstation (16), die eine Anzeigevorrichtung (14), die konfiguriert ist, um physiologische Informationen anzuzeigen, und einen Eingang (22), der konfiguriert ist, um eine oder mehrere Eingaben von einem Arzt zu empfangen, beinhaltet;

einen oder mehrere Computerprozessoren (18), die mit der Arbeitsstation (16) verbunden sind, wobei der eine oder die mehreren Computerprozessoren (18) zu Folgendem konfiguriert sind:

Erhalten physiologischer Informationen; Erstellen und anzeigen einen vorläufigen Bericht auf der Anzeigevorrichtung (14); Empfangen und Anzeigen von Befundcodes (40) aus der Eingabe (22), die von dem Arzt zum Aufnehmen in den vorläufigen Bericht ausgewählt werden;

Erstellen eines oder mehrerer vorgeschlagener Befundcodes (48) unter Verwendung der erhaltenen physiologischen Informationen durch mindestens eine regelbasierte Implementierung, bei der ein Satz von Hintergrundregeln für ein Machen von Vorschlägen verwendet wird, wobei der Satz von Regeln die Korrelationen zwischen bereits vorhandenen Befundcodes (40) modelliert, und eine datenbasierte Implementierung, bei der Statistiken oder andere Arten von Werten, die aus einer Datenbank früherer Berichte abgeleitet werden, für ein Machen von Vorschlägen verwendet werden, indem retrospektive physiologische Informationen untersucht werden, um die ähnlichsten früheren Sätze von Befundcodes (40) und den wahrscheinlichsten nächsten eingegebenen Befundcode zu bestimmen; Anzeigen des einen oder der mehreren vorgeschlagenen Befundcodes, aus denen der Arzt wählen kann; Erhalten der Auswahl eines oder mehrerer der angezeigten Befundcodes durch einen Arzt; und

Aktualisieren der angezeigten Befundcodes auf der Anzeigevorrichtung (14) einschließlich eines Erstellens und Anzeigens eines narrativen Berichts (56) basierend auf mindestens einem der von dem Arzt ausgewählten Befundcodes (40, 48) und physiologischen Informationen.

2. Gerät (10) nach Anspruch 1, das ferner mindestens einen Speicher (20) beinhaltet, der konfiguriert ist, um Befundcodes (48) zu speichern.

3. Gerät (10) nach Anspruch 1, wobei die Anzeige (12A) Folgendes umfasst:

ein strukturiertes Berichtsfeld (26); und
eine Befundcode-Vorschlagstafel (28A/28B).

4. Gerät (10) nach Anspruch 3, wobei die Befundcode-Vorschlagstafel (28A/28B) entweder eine persistente Befundcode-Vorschlagstafel (28A) oder eine transiente Befundcode-Vorschlagstafel (28B) beinhaltet.

5. Gerät (10) nach Anspruch 3, wobei die Befundcode-Vorschlagstafel (28A/28B) Folgendes beinhaltet:

einen oder mehrere vorgeschlagene Befundcodes (48); und
eine oder mehrere Annahme-Schaltflächen (52), die dem einen oder den mehreren vorgeschlagenen Befundcodes (48) entsprechen, mit denen der Arzt einen oder mehrere der vorgeschlagenen Befundcodes auswählt.

6. Gerät (10) nach Anspruch 1, wobei der eine oder die mehreren vorgeschlagenen Befundcodes (48) eine Codekomponente (48A) und eine Textkomponente (48B) beinhalten.

7. Gerät (10) nach Anspruch 1, wobei der eine oder die mehreren vorgeschlagenen Findungscodes (48) mindestens einen binären Anweisungsfindungscode, einen Multiple-Choice-Anweisungsbefundcode und einen Messanweisung-Befundcode beinhalten.

8. Gerät (10) nach Anspruch 3, wobei das strukturierte Berichtsfeld (26) Folgendes umfasst:

ein oder mehrere anatomische Untermenüs (32);
ein oder mehrere Aspekt-Untermenüs (36);
ein oder mehrere Dropdown-Menüs;
eine oder mehrere vorgeschlagene Schaltflächen für Befundcode-Erstellung (34); und
einen oder mehrere angenommene Befundcodes (40).

9. Gerät (10) nach Anspruch 1, wobei der eine oder die mehreren Prozessoren (18) konfiguriert sind, um physiologische Informationen zu erhalten, die mindestens eines von physiologischen Bildgebungsinformationen des Patienten, Patientenaufzeichnungen, Patientenbefundcodes (48), strukturierte Patientenberichte (58) und gespeicherte Befundcode-Muster beinhalten.

10. Vorrichtung (10) nach Anspruch 3, wobei die Anzeige (12A/62A) ferner ein Bildfenster (24) umfasst.

11. Vorrichtung (10) nach Anspruch 10, wobei der Vorbericht ein strukturierter Bericht ist, der eine oder mehrere anatomische Zusammenfassungen (60) enthält; wobei die anatomischen Zusammenfassungen (60) die Textkomponenten (48A) der ausgewählten Befundcodes (40) aus einem strukturierten Befundpanel (26) enthalten.

12. Computerimplementiertes Verfahren zur Anleitung eines Arztes, nicht behandelte Aspekte eines Berichts zu behandeln, wobei das Verfahren Folgendes umfasst:

Empfang (S102) physiologischer Informationen an einem Arbeitsplatz (10);
Generierung (S104) und Berichts auf einem Anzeigegerät (14) unter Verwendung eines oder mehrerer Prozessoren (18);
Empfang und Anzeige von Befundcodes (40) aus dem Eingang (22), der vom Arzt für die Aufnahme in den Vorbericht ausgewählt wurde;
Generierung (S106) eines oder mehrerer vorgeschlagener Befundcodes (48) unter Verwendung der empfangenen physiologischen Informationen durch mindestens eine regelgesteuerte Implementierung; in dem eine Reihe von Hintergrundregeln für die Unterbreitung von Vorschlägen verwendet wird, die Regelsammlung zur Modellierung der Korrelationen zwischen bereits vorhandenen Befundcodes (40) und einer datengesteuerten Implementierung; in dem aus einer Datenbank mit früheren Berichten abgeleitete Statistiken oder andere Arten von Werten verwendet werden,
um Vorschläge zu unterbreiten, indem retrospektive physiologische Informationen untersucht werden, um die ähnlichsten vorherigen Befundcodes (40) und den wahrscheinlichsten nächsten eingegebenen Befundcode zu bestimmen;
Anzeige (S106) eines oder mehrerer vorgeschlagener Befundcodes (48), die das Klinikteam mit einem oder mehreren Prozessoren (18) auswählen kann; Empfang der Auswahl eines oder mehrerer der angezeigten Befundcodes durch das Klinikteam Von einer Benutzereingabe (22); und Aktualisierung der angezeigten Befundcodes, einschließlich Erstellung und Anzeige eines Befundberichts (56) auf der Grundlage eines oder mehrerer vom Arzt ausgewählter Befundcodes und physiologischer Informationen.

13. Verfahren (S100) nach Anspruch 12, wobei die Generierung der vorgeschlagenen Befundcodes (48) die Generierung einer Codekomponente (48B) und einer Textkomponente umfasst (48A).

14. Nicht-transitorisches computerlesbares Medium (20), das Software zum Steuern eines oder mehrerer Prozessoren (18) zum Ausführen des Verfahrens nach Anspruch 12 trägt.

**Revendications**

1. Appareil de rapport structuré guidé (10) pour guider un clinicien pour traiter des aspects non adressés d'un rapport, l'appareil comprenant:

   une station de travail (16) comprenant un dispositif d'affichage (14) configuré pour afficher des informations physiologiques, et une entrée (22) configurée pour recevoir une ou plusieurs entrées d'un clinicien; un ou plusieurs processeurs informatiques (18) connectés au poste de travail (16), le ou les processeurs informatiques (18) étant configurés pour:

   recevoir des informations physiologiques; générer et afficher un rapport préliminaire sur le dispositif d'affichage (14);
   recevoir et afficher des codes de découverte (40) à partir de l'entrée (22) sélectionnée par le clinicien pour inclusion dans le rapport préliminaire;
   générer un ou plusieurs codes de découverte suggérés (48) à l'aide des informations physiologiques reçues, par au moins une implémentation pilotée par des règles, dans laquelle un ensemble de règles d'arrière-plan est utilisé pour faire des suggestions, l'ensemble de règles modélisant les corrélations entre les pré- codes de découverte existants (40) et une implémentation basée sur les données, dans laquelle des statistiques ou d'autres types de valeurs dérivées d'une base de données de rapports antérieurs sont utilisés pour faire des suggestions en examinant des informations physiologiques rétrospectives afin de déterminer les ensembles de codes de découverte antérieurs les plus similaires (40) et le prochain code de découverte probablement saisi; afficher le ou les codes de recherche suggérés parmi lesquels le clinicien peut sélectionner; recevoir la sélection d'un ou plusieurs des codes de découverte affichés par un clinicien; et mettre à jour les codes de découverte affichés sur le dispositif d'affichage (14) comprenant la génération et l'affichage d'un rapport narratif (56) basé sur au moins l'un des codes de découverte sélectionnés par le clinicien (40, 48) et des informations physiologiques.

2. Appareil (10) selon la revendication 1, comprenant en outre au moins une mémoire (20) configurée pour stocker des codes de découverte (48).

3. Appareil (10) selon la revendication 1, dans lequel l'affichage (12A) comprend:

   un panneau de rapport structuré (26); et
   un panneau de suggestion de code de recherche (28A/28B).

4. Appareil (10) selon la revendication 3, dans lequel le panneau de suggestion de code de découverte (28A/28B) comprend l'un parmi un panneau de suggestion de code de découverte persistant (28A) et un panneau de suggestion de code de découverte transitoire (28B).

5. Appareil (10) selon la revendication 3, dans lequel le panneau de suggestion de code de recherche (28A/28B) comprend:

   un ou plusieurs codes de recherche suggérés (48); et
   un ou plusieurs boutons d'adoption (52) correspondant au ou aux codes de découverte suggérés (48) par lesquels le clinicien sélectionne un ou plusieurs des codes de découverte suggérés.

6. Appareil (10) selon la revendication 1, dans lequel le ou les codes de recherche suggérés (48) comprennent un composant de code (48A) et un composant textuel (48B) .

7. Appareil (10) selon la revendication 1, dans lequel le ou les codes de recherche suggérés (48) comprennent au moins l'un d'un code de recherche d'instruction binaire, d'un code de recherche d'instruction à choix multiples et d'un code de recherche d'instruction de mesure.

8. Appareil (10) selon la revendication 3, dans lequel le panneau de rapport structuré (26) comprend:

   un ou plusieurs sous-menus anatomiques (32);
   un ou plusieurs sous-menus d'aspect (36);
   un ou plusieurs menus déroulants;
   un ou plusieurs boutons de génération de code de recherche suggéré (34); et

un ou plusieurs codes de découverte adoptés (40).

9.  Appareil (10) selon la revendication 1, dans lequel le ou les processeurs (18) sont configurés pour recevoir des informations physiologiques comprenant au moins l'une parmi des informations d'imagerie physiologique du patient, des dossiers de patient, des codes de recherche de patient (48), des rapports structurés de patient (58) et des modèles de code de recherche stockés.

10. Appareil (10) selon la revendication 3, dans lequel l'affichage (12A/62A) comprend en outre un panneau d'imagerie (24).

11. Appareil (10) selon la revendication 10, dans lequel le rapport préliminaire est un rapport structuré comprenant un ou plusieurs résumés anatomiques (60); dans lequel les résumés anatomiques (60) comprennent les composants textuels (48A) des codes de découverte sélectionnés (40) à partir d'un panneau de rapport structuré (26).

12. Procédé mis en oeuvre par ordinateur pour guider un clinicien afin de traiter les aspects non traités d'un rapport, le procédé comprenant:

>   recevoir (S102) des informations physiologiques à un poste de travail (10);
>   générer (S104) et afficher un rapport préliminaire sur un dispositif d'affichage (14) en utilisant un ou plusieurs processeurs (18);
>   recevoir et afficher des codes de découverte (40) à partir de l'entrée (22) sélectionnée par le clinicien pour inclusion dans le rapport préliminaire;
>   générer (S106) un ou plusieurs codes de découverte suggérés (48) à l'aide des informations physiologiques reçues, par au moins l'une d'une mise en oeuvre guidée par des règles, dans laquelle un ensemble de règles d'arrière-plan est utilisé pour faire des suggestions,
>   l'ensemble de règles modélisant les corrélations entre des codes de découverte préexistants (40) et une implémentation basée sur les données, dans laquelle des statistiques ou d'autres types de valeurs dérivées d'une base de données de rapports antérieurs sont utilisés pour faire des suggestions en examinant des informations physiologiques rétrospectives afin de déterminer les ensembles antérieurs de découvertes les plus similaires les codes (40) et le prochain code de découverte entré probablement;
>   afficher (S106) le ou les codes de découverte suggérés (48) pour que le clinicien sélectionne parmi l'utilisation d'un ou de plusieurs processeurs (18);
>   recevoir la sélection d'un clinicien d'un ou plusieurs des codes de découverte affichés à partir d'une entrée utilisateur (22); et
>   la mise à jour des codes de découverte affichés comprenant la génération et l'affichage d'un rapport narratif (56) basé sur un ou plusieurs des codes de découverte sélectionnés par le clinicien et des informations physiologiques.

13. Procédé (S100) selon la revendication 12, dans lequel la génération des codes de recherche suggérés (48) comprend la génération d'un composant de code (48B) et d'un composant textuel (48A).

14. Support lisible par ordinateur non transitoire (20) portant un logiciel pour contrôler un ou plusieurs processeurs (18) pour exécuter le procédé selon la revendication 12.

FIG. 1

FIG. 2

EP 3 271 844 B1

**FIG. 3**

FIG. 4

EP 3 271 844 B1

26

| Information | Measure | Score | Interpret | Comments | ← 30

| LV | RV | Atria | MV | TV |
| AV | PV | Great Vessels | PE | ← 32

Finding Code:

42 →

[ ]  [ Run Auto Pop ]  [ Run Rules ] ← 34

ac00: LV Size/Shape

40 → **LV-0061 Grossly normal size**

← 36

ac00: LV Thrombus/VSD

40 → LV-0125 Apical thrombus, moderate size
40 → **LV-0129 Thrombus appears mobile**

ac00: LV Thickness

40 → LV-0066 Global thinning

← 36

ac00: LV Function

← 36

ac00: LV Wall Motion

← 36

# FIG. 5

EP 3 271 844 B1

28B

44

Based on already-inserted FCs, the following FCs are suggested: ✕

⊕ LV601.5 Apical thrombus noted after injection of echocardiographic contrast.

⊕ SU-0076 No significant valvular heart disease.

52   48B   48A   50   48   46

FIG. 6

Based on already-inserted FCs, the following FCs are suggested:

⊕ LV601.5 Apical thrombus noted after injection of echocardiographic contrast.

⊕ SU-0076 No significant valvular heart disease.

FIG. 7

56

Preliminary

Study ID: 10

Name: RuleGen Demo4          Study Date: 10/17/2013  10:14 AM
MRN: 4                                        Gender: Female
DOB: 01/01/1985 (M/d/yyyy)
Age: 28 yrs

Interpretation Summary

Left Ventricle
The left ventricle is grossly normal size. There is a moderate size apical thrombus. The thrombus appears mobile.
There is global thinning of the left ventricular walls.

Reading Physician:
            11/27/2013 09:33 AM                    60

58

## FIG. 8

62A

56    26    28A

Preliminary    Study ID: 10

Information | Measure | Score | Interpret | Comments
LV | RV | Atria | MV | TV
AV | PV | Great Vessels | PE

Based on already-inserted FCs, the following FCs are suggested:

LV601.5 Apical thrombus noted after injection of echocardiographic contrast.

SU-0076 No significant valvular heart disease.

Finding Code: | Run Auto Pop | Run Rules

ac00: LV Size/Shape
LV-0061 Grossly normal size

ac00: LV Thrombus/VSD
LV-0125 Apical thrombus, moderate size
LV-0129 Thrombus appears mobile

ac00: LV Thickness
LV-0065 Global thinning

ac00: LV Function

ac00: LV Wall Motion

Name: RuleGen Demo4       Study Date: 10/17/2013  10:14 AM
MRN: 4                    Gender: Female
DOB: 01/01/1985 (M/d/yyyy)
Age: 28 yrs

Interpretation Summary

Left Ventricle
The left ventricle is grossly normal size. There is a moderate size apical thrombus. The thrombus appears mobile. There is global thinning of the left ventricular walls.

Reading Physician: —————————————————————
        11/27/2013 09:33 AM

FIG. 9

EP 3 271 844 B1

62A

Preliminary

Study ID: 10

| Information | Measure | Score | Interpret | Comments |
| LV | RV | Aorta | MV | TV |
| AV | PV | Great Vessels | PE |

Based on already-inserted FCs, the following FCs are suggested:

○ LV601.5 Apical thrombus noted after injection of echocardiographic contrast.

○ SU-0076 No significant valvular heart disease.

Finding Code: [ ] [Run Auto Pop] [Run Rules]

ac00: LV Size/Shape

ac00: LV Thrombus/VSD
LV-0125 Apical thrombus, moderate size

ac00: LV Thickness
LV-0066 Global thinning

ac00: LV Function

ac00: LV Wall Motion

Name: RuleGen Demo4          Study Date: 10/17/2013  10:14 AM
MRN: 4                       Gender: Female
DOB: 01/01/1985 (M/d/yyyy)
Age: 28 yrs

Interpretation Summary

Left Ventricle
The left ventricle is grossly normal size. There is a moderate size apical thrombus. The thrombus appears mobile. There is global thinning of the left ventricular walls.

Reading Physician: —————————————————————
          11/27/2013 09:33 AM

14

FIG. 10

S100

S102 — Receive physiological information

S104 — Generate reporting and suggestion panels in a display

S106 — Generate and display FC suggestions in a display

S108A — Receive input from an input device?

Yes → Update the display — S110

No

S108B — Receive new physiological information?

Yes →

FIG. 11

23

**EP 3 271 844 B1**

**REFERENCES CITED IN THE DESCRIPTION**

**Patent documents cited in the description**

- US 2008004505 A